# EUROPEAN PATENT SPECIFICATION

(11) **EP 3 065 772 B1**
(45) Date of publication and mention of the grant of the patent: **24.07.2024**
(21) Application number: 14859634.9
(22) Date of filing: 05.11.2014
(51) Int. Cl.: A61K 39/00, A61K 39/39, A61K 39/395, A61K 38/20, C07K 16/28, A61N 5/10, A61P 35/04, A61P 43/00, A61K 35/15, A61P 35/00, A61P 35/02, A61P 37/04

(54) **COMBINATIONS OF CHECKPOINT INHIBITORS AND THERAPEUTICS TO TREAT CANCER**
KOMBINATIONEN AUS CHECKPOINT-INHIBITOREN UND THERAPEUTIKA ZUR BEHANDLUNG VON KREBS
COMBINAISONS D'INHIBITEURS DE POINT DE CONTRÔLE ET D'AGENTS THÉRAPEUTIQUES POUR TRAITER UN CANCER

(30) Priority: 05.11.2013 US 201361900355 P; 05.11.2013 US 201361900309 P
(43) Date of publication of application: 14.09.2016
(73) Proprietor: Cognate Bioservices, Inc., Hanover, MD 21076 (US); NorthWest Biotherapeutics, Inc., Bethesda, MD 20814 (US); The Regents of the University of California, Oakland, CA 94607 (US); REVIMMUNE, INC., Hanover MD 21076 (US)
(72) Inventor: BOSCH, Marnix, Leo, Clyde Hill, WA 98004 (US); GANJEI, James, Kelly, Bethesda, MD 20814 (US); POWERS, Linda, F., Bethesda, MD 20814 (US); LIAU, Linda, M., Los Angeles, CA 90077 (US); PRINS, Robert, M., Pacific Palisades, CA 90272 (US)
(74) Representative: Forresters IP LLP
(86) International application number: PCT/US2014/064133
(87) International publication number: WO 2015/069770

(56) References cited:
- WO-A1-2005/092371
- WO-A2-2012/177624
- US-A1- 2013 004 483
- US-B2- 8 142 778
- KAROLINA PALUCKA ET AL: "Dendritic-cell-based therapeutic cancer vaccines", IMMUNITY, 25 July 2013 (2013-07-25), United States, pages 38 - 48, XP055308708, Retrieved from the Internet <URL:https://www.ncbi.nlm.nih.gov/pmc/articles/PMC3788678/pdf/nihms511491.pdf> DOI: 10.1016/j.immuni.2013.07.004
- GE YAN ET AL: "Blockade of PD-1/PD-L1 immune checkpoint during DC vaccination induces potent protective immunity against breast cancer in hu-SCID mice", CANCER LETTERS, NEW YORK, NY, US, vol. 336, no. 2, 21 March 2013 (2013-03-21), pages 253 - 259, XP028575480, ISSN: 0304-3835, DOI: 10.1016/J.CANLET.2013.03.010
- VANNEMAN ET AL.: "Combining immunotherapy and targeted therapies in cancer treatment", NATURE REVIEWS CANCER, vol. 12, 1 April 2012 (2012-04-01), pages 237 - 251, XP055045378
- FEHNIGER ET AL.: "Interleukin 15: biology and relevance to human disease", BLOOD, vol. 97, no. 1, 1 January 2001 (2001-01-01), pages 14 - 32, XP002902996
- PARDOLL ET AL.: "The blockade of immune checkpoints in cancer immunotherapy", NATURE REVIEWS CANCER, vol. 12, 1 April 2012 (2012-04-01), pages 252 - 264, XP055339916
- HODI ET AL.: "Improved Survival with Ipilimumab in Patients with Metastatic Melanoma", N ENGL J MED, vol. 363, 19 August 2010 (2010-08-19), pages 711 - 723, XP055015428

## Description

### CROSS REFERENCE TO RELATED APPLICATIONS

This application claims the benefit of priority under 35 USC §119(e) to U.S. Application Ser. 61/900,309, filed November 5, 2013 and U.S. Application Ser. 61/900,355, filed on November 5, 2013.

### BACKGROUND OF THE INVENTION

### FIELD OF THE INVENTION

The invention relates to a combination of checkpoint inhibitors and therapeutics for use in a method of treating a glioma or initiating, enhancing, or prolonging an anti-glioma response in a subject in need thereof.

### BACKGROUND INFORMATION

Increasing evidence suggests that the ability to outsmart the body's immune response represents a hallmark of tumor development. As such, researchers have begun to look at ways to reinstate the immune response with targeted agents, essentially indirectly treating cancer by treating the immune system. One particularly promising strategy for doing this is to target so-called immune checkpoints, which act as the off-switch on the T cells of the immune system.

Checkpoint inhibitor therapies, which 'unblock' an existing immune response or which unblock the initiation of an immune response are very effective at treating cancer in a subgroup of subjects. However, the subgroup of subjects is relatively small population constituting only approximately 25% of the cancer subject population (i.e., the "responding subject population"). Accordingly, while checkpoint inhibitors are extremely effective at treating cancers in the responding subject population, approximately 75% of cancer subjects will not respond to the therapy. In addition, even in the responding population the response is not always complete or optimal.

Since many of the immune checkpoints are regulated by interactions between specific receptor and ligand pairs, monoclonal antibodies or other agents can be used to block this interaction and prevent immunosuppression. The two checkpoint receptors that have received the most attention in recent years are CTLA-4 and PD-1.

CTLA-4, PD-1 and its ligands are members of the CD28-B7 family of co-signaling molecules that play important roles throughout all stages of T-cell function and other cell functions. The PD-1 receptor is expressed on the surface of activated T cells (and B cells) and, under normal circumstances, binds to its ligands (PD-L1 and PD-L2) that are expressed on the surface of antigen-presenting cells, such as dendritic cells or macrophages. This interaction sends a signal into the T cell and essentially switches it off or inhibits it. Cancer cells take advantage of this system by driving high levels of expression of PD-L1 on their surface. This allows them to gain control of the PD-1 pathway and switch off T cells expressing PD-1 that may enter the tumor microenvironment, thus suppressing the anticancer immune response.

A first-in-class immunotherapy, ipilimumab (Yervoy), a monoclonal antibody that targets cytotoxic T-lymphocyte-associated antigen 4 (CTLA-4) on the surface of T cells, was approval for the treatment of melanoma. Now, a new targeted immunotherapy aimed at the programmed death-1 (PD-1) T-cell receptor or its ligand (PD-L1 or PD-L2) may prove to be more effective and even safer than ipilimumab. Additional checkpoint targets may also prove to be effective, such as TIM-3, LAG-3, various B-7 ligands, CHK 1 and CHK2 kinases, BTLA, A2aR, and others.

Currently, at least seven checkpoint inhibitor agents are in clinical trials. Among them are monoclonal anti-PD-1 antibodies, both fully human and humanized, as well as a fully human anti-PD-L1 antibody and a fusion protein combining the extracellular domain of PD-L2 and IgG1. Each of these agents is designed to block the interaction between PD-1 and its ligands, and thus keep the T-cell (or other cell) on/off switch in the "on" position, although they each have slightly different mechanisms of action.

Another strategy for the treatment of cancer is to combine a checkpoint inhibitor with a therapeutic. Biologic therapeutics, including antibodies and vaccines, have been proven to be effective in the treatment of cancer. The combination of a checkpoint inhibitor and a therapeutic (e.g., biologic) may enhance or prolong an anti-tumor response in a subject. Further, the administration of a biologic therapeutic with a checkpoint inhibitor may enhance or prolong the effects of the checkpoint inhibitor, enable a subject to respond to a checkpoint inhibitor, or enable the reduction of the toxicity or the dose of a checkpoint inhibitor.

GE YAN et al: "Blockade of PD-1/PD-L1 immune checkpoint during DC vaccination induces potent protective immunity against breast cancer in hu-SCID mice" Cancer Lett. (2013) 336(2):253-9 appears to disclose DC vaccination in a breast tumor-bearing hu-SCID mouse model.

Accordingly, there is a need to develop methods and combination therapies to initiate or enhance the effectiveness of the checkpoint inhibitors in both the nonresponding subject population and the responding subject population. The present invention discloses use in methods to initiate, enable, enhance or improve an anti-tumor immune response to subsequently enable, enhance or improve the subject's or tumor response to checkpoint inhibitors.

### SUMMARY OF THE INVENTION

The present disclosure arises at least in part from the seminal recognition that a combination treatment regimen including one or more cycles and/or doses of a checkpoint inhibitor and a therapeutic, either sequentially, in either order, or substantially simultaneously, can be more effective in treating cancer in some subjects and/or can initiate, enable, increase, enhance or prolong the activity and/or number of immune cells, or a medically beneficial response by a tumor.

The invention is defined in the claims. The present invention provides a therapeutic agent in combination with an agent that is a checkpoint inhibitor for use in a method of treating a glioma or initiating, enhancing, or prolonging an anti-glioma response in a subject in need thereof comprising administering to the subject the therapeutic agent in combination with the agent that is a checkpoint inhibitor. The checkpoint inhibitor is a monoclonal antibody, a humanized antibody, a fully human antibody or a combination thereof and inhibits a checkpoint protein which isPDL1 orPD1 or a combination thereof. The therapeutic agent is a glioma lysate-pulsed dendritic cell (DC) vaccine.

In an embodiment, the dendritic cell vaccine is composed of autologous dendritic cells and/or allogenic dendritic cells. In an embodiment, the autologous or allogenic dendritic cells are loaded with glioma lysate prior to administration to the subject.

The invention also provides a therapeutic agent in combination with an agent that is a checkpoint inhibitor for use in a method of treating glioma or initiating, enhancing, or prolonging an anti-glioma response in a subject in need thereof comprising administering to the subject the therapeutic agent in combination with the agent that is a checkpoint inhibitor, wherein the therapeutic agent is a dendritic cell vaccine, wherein the dendritic cells of the vaccine are partially matured, and wherein the checkpoint inhibitor is selected from the group consisting of a monoclonal antibody, a humanized antibody and a fully human antibody or a combination thereof and inhibits a checkpoint protein selected from the group consisting of PD-1 and PDL1 or a combination thereof and wherein the partially mature dendritic cells are autologous or allogenic dendritic cells which pick up glioma biomarkers in situ. In a further embodiment, the checkpoint inhibitor and the therapeutic are administered simultaneously or sequentially, in either order. In an additional aspect, the therapeutic is administered prior to the checkpoint inhibitor and the checkpoint inhibitor is a PD-1 inhibitor.

In a further embodiment, the glioma is malignant glioblastoma.

In a further embodiment, use in the method further comprises administering a chemotherapeutic agent, targeted therapy or radiation to the subject either prior to, simultaneously with, or after treatment with the combination therapy.

In a further embodiment, the subject has a glioma and the method enhances or prolongs the effects of the checkpoint inhibitor, or enables a subject to respond to the checkpoint inhibitor, or enables the toxicity or the dose of the checkpoint inhibitor to be reduced.

### BRIEF DESCRIPTION OF THE DRAWINGS

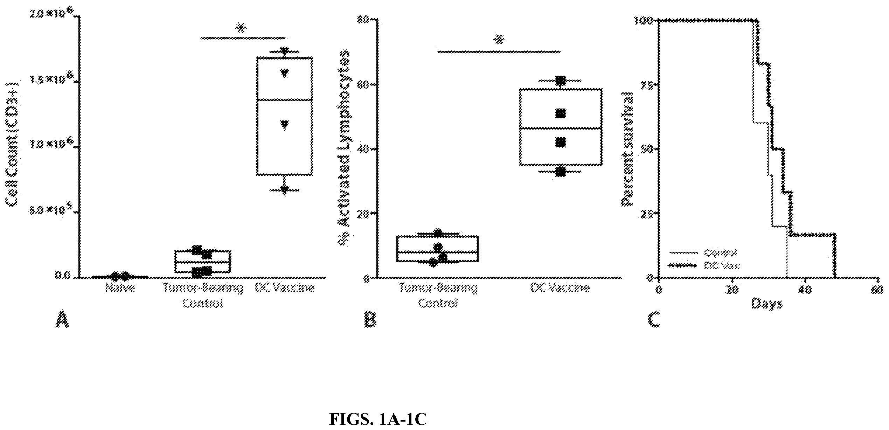

Figures 1A-C show that the DC vaccine promotes activated lymphocytic infiltration without therapeutic benefit in well-established gliomas. (A) DC vaccine promoted significant tumor infiltration of lymphocytes (CD3+) over controls. (B) A significant proportion of these cells were activated lymphocytes (CD3+ CD8+ CD25+). (C) No survival benefit between DC vaccine and control groups was noted.

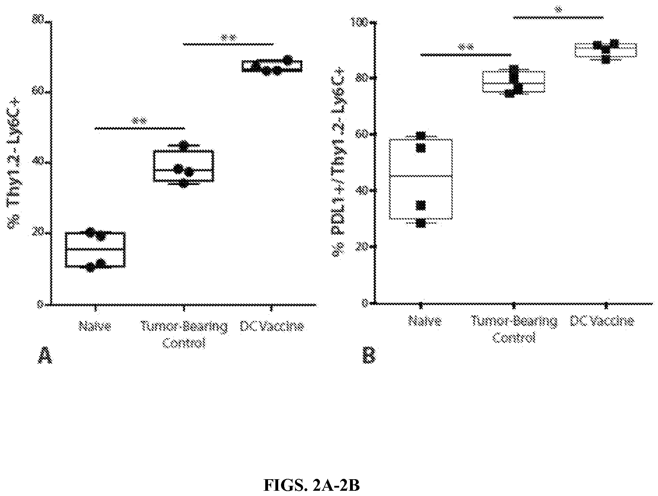

Figures 2A-B show that there is an increased accumulation of inhibitory myeloid cells in the tumor microenvironment after DC vaccine. (A) Tumor-bearing controls showed an increase in tumor-infiltrating myeloid cells (Thy1.2- Ly6C+). This population was significantly greater in mice treated with DC vaccine. (B) A significant percentage of these myeloid cells expressed PD-L1 (Thy1.2- Ly6C+ PD-L1+).

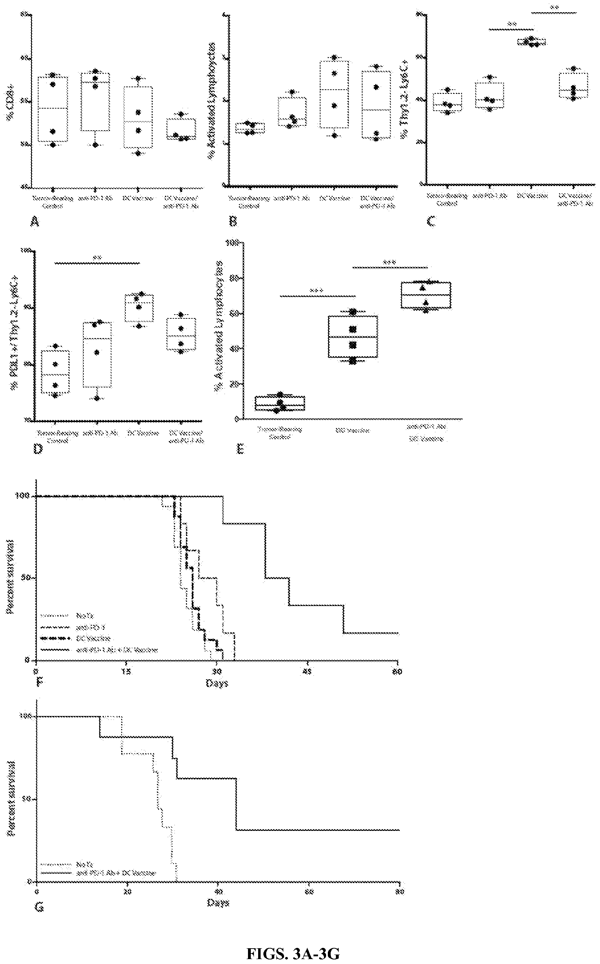

Figures 3A-G show that blockade of PD-1 prevents the accumulation of inhibitory myeloid cells and promotes activated lymphocytic infiltration with therapeutic benefit. (A) There was no difference in CD8+ population in lymph nodes between DC vaccinated and DC vaccine mice treated with adjuvant anti-PD-1 Ab (DC Vaccine/ anti-PD-1 Ab). (B) A minor population of activated CD8+ CD25+ T cells was found in the lymph nodes of each treatment group and was not statistically distinct. (C, D) The inhibitory myeloid population was significantly reduced in the DC vaccine/anti-PD-1 treated mice when compared to groups receiving DC vaccination alone. (E) There was a comparable population of activated cytotoxic tumor-infiltrating lymphocytes in DC vaccine/anti-PD-1 treated mice comparable to DC vaccination alone. (F) Combination DC vaccine/anti-PD-1 treatment showed significant survival benefit. (G) Surviving DC vaccine/anti-PD-1 treated mice from (F) were re-challenged with contralateral intracranial implant of GL261 murine glioma on day 75 without additional treatments. Significant survival benefit was noted when compared to control mice.

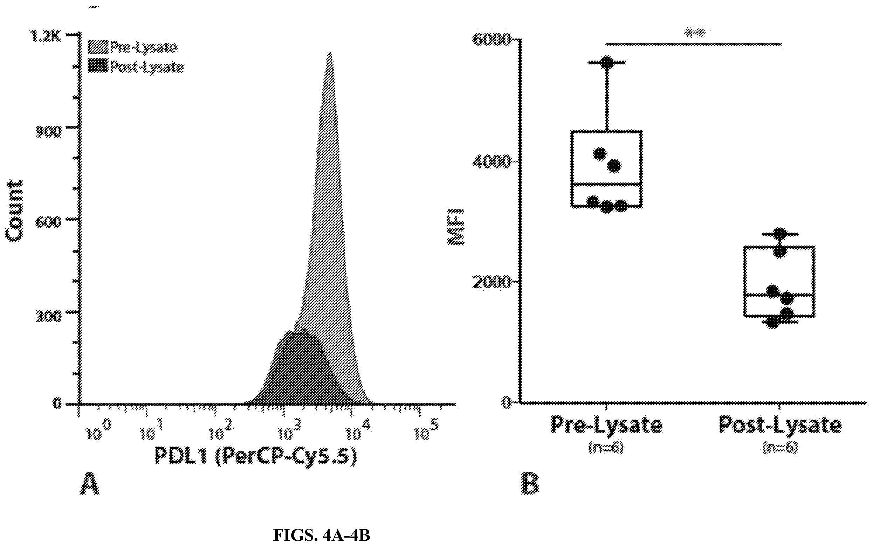

Figures 4A-B show that tumor lysate-pulsed dendritic cells down-regulate expression of PD-L1. (A) Representative histogram depicting change in PD-L1 expression pre and post-lysate pulsing. (B) Dendritic cells pulsed 24h with tumor lysate and then analyzed for PD-L1 expression. The median fluorescence intensity (MFI) of PD-L1 expression is graphed.

### DETAILED DESCRIPTION OF THE INVENTION

The present disclosure arises at least in part from the seminal recognition that a combination treatment regimen including one or more cycles and/or doses of a checkpoint inhibitor and a therapeutic, either sequentially, in either order, or substantially simultaneously, can be more effective in treating cancer in some subjects and/or can initiate, enable, increase, enhance or prolong the activity and/or number of immune cells, or a medically beneficial response by a tumor.

It is to be understood that the terminology used herein is for purposes of describing particular embodiments only, and is not intended to be limiting, since the scope of the present invention is defined in the claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

As used herein, the term "therapeutic" of "therapeutic agent" refers to any medicinal product that produces a therapeutic response in a subject.

As used herein, the term "biologic therapeutic" or "biopharmaceutical" refers to any medicinal product manufactured in or extracted from biological sources. Biopharmaceuticals are distinct from chemically synthesized pharmaceutical products. Examples of biopharmaceuticals include vaccines, blood or blood components, allergenics, somatic cells, gene therapies, tissues, recombinant therapeutic proteins, including antibody therapeutics and fusion proteins, and living cells. Biologics can be composed of sugars, proteins or nucleic acids or complex combinations of these substances, or may be living entities such as cells and tissues. Biologics are isolated from a variety of natural sources - human, animal or microorganism - and may be produced by biotechnology methods and other technologies. Specific examples of biologic therapeutics include, but are not limited to, immunostimulatory agents, T cell growth factors, interleukins, antibodies, fusion proteins and vaccines, such as cancer vaccines.

As used herein, the term "antibody" refers to an immunoglobulin or a part thereof, and encompasses any polypeptide comprising an antigen-binding site regardless of the source, species of origin, method of production, and characteristics. Antibodies may be comprised of heavy and/or light chains or fragments thereof. Antibodies or antigen-binding fragments, variants, or derivatives thereof of the disclosure include, but are not limited to, polyclonal, monoclonal, multispecific, human, humanized, primatized, or chimeric antibodies, single chain antibodies, epitope-binding fragments, e.g., Fab, Fab' and F(ab')₂, Fd, Fvs, single-chain Fvs (scFv), single-chain antibodies, disulfide-linked Fvs (sdFv), fragments comprising either a VL or VH domain, fragments produced by a Fab expression library, and anti-idiotypic (anti-Id) antibodies. ScFv molecules are known in the art and are described, e.g., in U.S. Pat. No. 5,892,019. Immunoglobulin or antibody molecules of the invention can be of any type (e.g., IgG, IgE, IgM, IgD, IgA, and IgY), class (e.g., IgG1, IgG2, IgG3, IgG4, IgA1 and IgA2) or subclass of immunoglobulin molecule.

Cell surface receptors are common targets for antibody therapies and include the epidermal growth factor receptor and HER2. Once bound to a cancer antigen, antibodies can induce antibody-dependent cell-mediated cytotoxicity, activate the complement system, prevent a receptor interacting with its ligand or deliver a payload of chemotherapy or radiation, all of which can lead to cell death. Antibodies approved as therapeutic agents include Rituxan^{®} (Rituximab); Zenapax^{®} (Daclizumab); Simulect^{®} (Basiliximab); Synagis^{®} (Palivizumab); Remicade^{®} (Infliximab); Herceptin^{®} (Trastuzumab); Mylotarg^{®} (Gemtuzumab ozogamicin); Campath^{®} (Alemtuzumab); Zevalin^{®} (Ibritumomab tiuxetan); Humira^{®} (Adalimumab); Xolair^{®} (Omalizumab); Bexxar^{®} (Tositumomab-I-131); Raptiva^{®} (Efalizumab); Erbitux^{®} (Cetuximab); Avastin^{®} (Bevacizumab); Tysabri^{®} (Natalizumab); Actemra^{®} (Tocilizumab); Vectibix^{®} (Panitumumab); Lucentis^{®} (Ranibizumab); Soliris^{®} (Eculizumab); Cimzia^{®} (Certolizumab pegol); Simponi^{®} (Golimumab); Ilaris^{®} (Canakinumab); Stelara^{®} (Ustekinumab); Arzerra^{®} (Ofatumumab); Prolia^{®} (Denosumab); Numax^{®} (Motavizumab); ABThrax^{®} (Raxibacumab); Benlysta^{®} (Belimumab); Yervoy^{®} (Ipilimumab); Adcetris^{®} (Brentuximab Vedotin); Perjeta^{®} (Pertuzumab); Kadcyla^{®} (Ado-trastuzumab emtansine); and Gazyva^{®} (Obinutuzumab).

As used herein, the term "fusion protein" refers to chimeric molecules which comprise, for example, an immunoglobulin antigen-binding domain with at least one target binding site, and at least one heterologous portion, i.e., a portion with which it is not naturally linked in nature. The amino acid sequences may normally exist in separate proteins that are brought together in the fusion polypeptide or they may normally exist in the same protein but are placed in a new arrangement in the fusion polypeptide. Fusion proteins may be created, for example, by chemical synthesis, or by creating and translating a polynucleotide in which the peptide regions are encoded in the desired relationship.

As used herein, the term "targeted therapy" refers to any therapeutic molecule which targets any aspect of the immune system.

As used herein, the term "cancer" refers to the broad class of disorders characterized by hyperproliferative cell growth, either in vitro (e.g., transformed cells) or in vivo. Conditions which can be treated or prevented by use in methods of the disclosure include, e.g., a variety of neoplasms, including benign or malignant tumors, a variety of hyperplasias, or the like. Use in the methods of the disclosure can achieve the inhibition and/or reversion of undesired hyperproliferative cell growth involved in such conditions. Specific examples of cancer include Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS--Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland'Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor.

As used herein, the term "improving survival" refers to an increase in lifespan or quality of life of a subject suffering from a cancer or proliferative disease. For example, improving survival also includes promoting cancer remission, preventing tumor invasion, preventing tumor reoccurrence, slowing tumor growth, preventing tumor growth, decreasing tumor size, and decreasing total cancer cell counts.

As used herein, the term "preventing a disorder" as used herein, is not intended as an absolute term. Instead, prevention, e.g., of a cancer, refers to delay of onset, reduced frequency of symptoms, reducing the likelihood that a subject exhibits symptoms associated with a disorder or acquires a disorder compared to similar subjects that do not receive at least one of the methods, compositions or treatments described herein, or reduced severity of symptoms associated with the cancer. Prevention therefore refers to a broad range of prophylactic measures that will be understood by those in the art. In some circumstances, the frequency and severity of symptoms is reduced to non-pathological levels, *e.g.,* so that the individual can delay invasive cancer treatment such as aggressive chemotherapies and surgery.

As used herein, the term "treating cancer" is not intended to be an absolute term. In some aspects, use in the methods of the invention seek to reduce the size of a tumor or number of cancer cells, cause a cancer to go into remission, or prevent growth in size or cell number of cancer cells. In some circumstances, treatment with the leads to an improved prognosis.

As used herein, the term "a subject in need of treatment" refers to an individual or subject that has been diagnosed with cancer or a cell proliferative disorder.

The terms "therapeutically effective", "therapeutically effective amount", "effective amount" or "in an amount effective" refers to a sufficient amount or dosage to promote the desired physiological response, such as but not limited to an amount or dosage sufficient to promote a T-cell response.

As used herein, the term "PD-1 antibodies" refers to antibodies that antagonize the activity and/or proliferation of lymphocytes by agonizing PD-1. The term "antagonize the activity" relates to a decrease (or reduction) in lymphocyte proliferation or activity that is at least about 10%, 20%, 30%, 40%, 50%, 60%, 70%, 80%, 90%, or more. The term "antagonize" may be used interchangeably with the terms "inhibitory" and "inhibit". PD-1-mediated activity can be determined quantitatively using T cell proliferation assays as described herein.

One aspect of the present disclosure provides antibodies that can act as agonists of PD-1, thereby modulating immune responses regulated by PD-1. In one embodiment, the anti-PD-1 antibodies can be novel antigen-binding fragments. Anti-PD-1 antibodies disclosed herein are able to bind to including human PD-1 and agonize PD-1, thereby inhibiting the function of immune cells expressing PD-1. In some embodiments, the immune cells are activated lymphocytes, such as T-cells, B-cells and/or monocytes expressing PD-1.

As used herein, the term "tumor response" refers to cellular responses including but not limited to triggering programmed cell death.

As used herein, the term "anti-tumor response" refers to an immune system response including but not limited to activating T-cells to attack an antigen or an antigen presenting cell.

As used herein, the term "initiating" refers to starting a first anti-tumor response or starting a second or "enhanced" anti-tumor response.

As used herein, the term "enabling" refers to the allowing a subject to respond or tumor cell to respond to a treatment disclosed herein, wherein the subject or tumor cell previously could not respond to the treatment or had a low response to the treatment.

As used herein, the term "enhancing" refers to allowing a subject or tumor cell to improve its ability to respond to a treatment disclosed herein. For example, an enhanced response may comprise an increase in responsiveness of 5%, 10%, 15%, 20%, 25%, 30%, 35%, 40%, 45%, 50%, 55%, 60%, 65%, 70%, 75%, 80%, 85%, 90%, 95% or 98% or more. As used herein, "enhancing" can also refer to enhancing the number of subjects who respond to a treatment such as a checkpoint inhibitor therapy. For example, an enhanced response may refer to a total percentage of subjects who respond to a treatment wherein the percentage is of 5%, 10%, 15%, 20%, 25%, 30%, 35%, 40%, 45%, 50%, 55%, 60%, 65%, 70%, 75%, 80%, 85%, 90%, 95% or 98% or more.

As used herein, the term "small molecule" refers to a low molecular weight (<900 daltons) organic compound that may help regulate a biological process, with a size on the order of 10⁻⁹ m. Most drugs are small molecules.

The present invention describes a novel combination treatment based on activating the adaptive immune resistance. The adaptive immune resistance mechanism implies that an agent that can block an induced immune-checkpoint protein, will be minimally effective because they will only work when there is a pre-existing anti-tumor immune response (i.e., activated T-cells). Patients who do not have pre-existing anti-tumor responses, the checkpoint inhibitors will not be effective. Accordingly, a combination treatment that can activate anti-tumor activity (i.e., an anti-tumor immune response) and inhibit the checkpoints is preferable because it would allow subjects who do not respond to either treatment alone to benefit from the combined treatment.

Dendritic cells (DCs) have been shown to coordinate T cell anti-tumor response and active vaccination strategies utilizing DCs to induce antitumor immunity in glioblastoma subjects have been tested. Multiple studies report significant and effective immune response following DC vaccine treatment. The possibility to further advance this immunotherapy with the adjuvant modulation of endogenous auto-regulatory mechanisms is of interest.

Dendritic cells are a diverse population of antigen presenting cells found in a variety of lymphoid and non-lymphoid tissues. (See Liu, Cell 106:259-62 (2001); Steinman, Ann. Rev. Immunol. 9:271-96 (1991)). Dendritic cells include lymphoid dendritic cells of the spleen, Langerhans cells of the epidermis, and veiled cells in the blood circulation. Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD11c, CD80, CD86, and are HLA-DR⁺, but are CD14⁻.

In contrast, monocytic dendritic cell precursors (typically monocytes) are usually CD14⁺. Monocytic dendritic cell precursors can be obtained from any tissue where they reside, particularly lymphoid tissues such as the spleen, bone marrow, lymph nodes and thymus. Monocytic dendritic cell precursors also can be isolated from the circulatory system. Peripheral blood is a readily accessible source of monocytic dendritic cell precursors. Umbilical cord blood is another source of monocytic dendritic cell precursors. Monocytic dendritic cell precursors can be isolated from a variety of organisms in which an immune response can be elicited. Such organisms include animals, for example, including humans, and non-human animals, such as, primates, mammals (including dogs, cats, mice, and rats), birds (including chickens), as well as transgenic species thereof. In certain embodiments, the monocytic dendritic cell precursors and/or immature dendritic cells can be isolated from a healthy subject or from a subject in need of immunostimulation, such as, for example, a cancer subject or other subject for whom cellular immunostimulation can be beneficial or desired (i.e., a subject having a bacterial or viral infection, and the like). Dendritic cell precursors and/or immature dendritic cells also can be obtained from an HLA-matched healthy individual for partial activation and administration to an HLA-matched subject in need of immunostimulation.

Methods of isolating and modifying dendritic cell precursors can be found in U.S. Pat. Pub. No. 20060057120.

Immune checkpoints regulate T cell function in the immune system. T cells play a central role in cell-mediated immunity. Checkpoint proteins interact with specific ligands which send a signal into the T cell and essentially switch off or inhibit T cell function. Cancer cells take advantage of this system by driving high levels of expression of checkpoint proteins on their surface which results in control of the T cells expressing checkpoint proteins on the surface of T cells that enter the tumor microenvironment, thus suppressing the anticancer immune response. As such, inhibition of checkpoint proteins would result in restoration of T cell function and an immune response to the cancer cells. Examples of checkpoint proteins include, but are not limited to CTLA-4, PDL1, PDL2, PD1, B7-H3, B7-H4, BTLA, HVEM, TIM3, GAL9, LAG3, VISTA, KIR, 2B4 (belongs to the CD2 family of molecules and is expressed on all NK, γδ, and memory CD8⁺ (αβ) T cells), CD160 (also referred to as BY55), CGEN-15049, CHK 1 and CHK2 kinases, A2aR and various B-7 family ligands.

Programmed cell death protein 1 (PD-1) is a 288 amino acid cell surface protein molecule is expressed on T cells and pro-B cells and plays a role in their fate/ differentiation. PD-1 has two ligands, PD-L1 and PD-L2, which are members of the B7 family. PD-L1 protein is upregulated on macrophages and dendritic cells (DC) in response to LPS and GM-CSF treatment, and on T cells and B cells upon TCR and B cell receptor signaling, whereas in resting mice, PD-L1 mRNA can be detected in the heart, lung, thymus, spleen, and kidney. PD-1 negatively regulates T cell responses.

PD-1 has been shown to be involved in regulating the balance between T cell activation and T cell tolerance in response to chronic antigens. For example, PD-1 expression has been extensively studied in HIV infection. During HIV1 infection, expression of PD-1 has been found to be increased in CD4+ T cells. It is thought that PD-1 up-regulation tied to T cell exhaustion (defined as a progressive loss of key effector functions) when T-cell dysfunction is observed in the presence of chronic antigen exposure as is the case in HIV infection. PD-1 up-regulation may also be associated with increased apoptosis in these same sets of cells during chronic viral infection (see Petrovas et al, (2009) J Immunol. 183 (2):1120-32).

PD-1 also plays a role in tumor-specific escape from immune surveillance. It has been demonstrated that PD-1 is highly expressed in tumor-specific cytotoxic T lymphocytes (CTLs) in both chronic myelogenous leukemia (CML) and acute myelogenous leukemia (AML). PD-1 is also up-regulated in melanoma infiltrating T lymphocytes (TILs) (see Dotti (2009) Blood 114 (8): 1457-58). Tumors have been found to express the PD-1 ligand (PDL-1 and PDL-2) which, when combined with the up-regulation of PD-1 in CTLs, may be a contributory factor in the loss in T cell functionality and the inability of CTLs to mediate an effective anti-tumor response. Researchers have shown that in mice chronically infected with lymphocytic choriomeningitis virus (LCMV), administration of anti-PD-1 antibodies blocked PD-1-PDL interaction and was able to restore some T cell functionality (proliferation and cytokine secretion), and lead to a decrease in viral load (Barber et al (2006) Nature 439 (9): 682-687).

Tumor cells themselves expresses PD-L1 and are thought to limit T cell responses via this mechanism. It has further been shown that inhibition of PD-1 results in expansion of effector T cells and restriction of T regulatory cell population in B16 melanoma models. Blockade of PD-1, CTLA-4, or IDO restores IL-2 production and allows for increased proliferation of CD8+ T cells present in the tumor microenvironment. It has been shown that anti-PDL1 treatment rescues and allows expansion of antigen-specific vaccine-generated CD8+ T cells to reject tumor. These data suggest that PD-1/PD-L1 axis regulates activated tumor-specific T cells.

Clinical trials in melanoma have shown robust anti-tumor responses with anti-PD-1 blockade. Significant benefit with PD-1 inhibition in cases of advanced melanoma, non-small-cell lung, prostate, renal-cell, and colorectal cancer have also been described. Studies in murine models have applied this evidence to glioma therapy. Anti-PD-1 blockade adjuvant to radiation promoted cytotoxic T cell population and an associated long-term survival benefit in mice with glioma tumor. A decrease in tumor-infiltrating Tregs and increased survival when combinatorial treatment of IDO, CTLA-4, and PD-L1 inhibitors was administered has been described.

There are several PD-1 inhibitors currently being tested in clinical trials. CT-01 1 is a humanized IgG1 monoclonal antibody against PD-1. A phase II clinical trial in subjects with diffuse large B-cell lymphoma (DLBCL) who have undergone autologous stem cell transplantation was recently completed. Preliminary results demonstrated that 70% of subjects were progression-free at the end of the follow-up period, compared with 47% in the control group, and 82% of subjects were alive, compared with 62% in the control group. This trial determined that CT-01 1 not only blocks PD-1 function, but it also augments the activity of natural killer cells, thus intensifying the antitumor immune response.

BMS 936558 is a fully human IgG4 monoclonal antibody targeting PD-1 agents under In a phase I trial, biweekly administration of BMS-936558 in subjects with advanced, treatment-refractory malignancies showed durable partial or complete regressions. The most significant response rate was observed in subjects with melanoma (28%) and renal cell carcinoma (27%), but substantial clinical activity was also observed in subjects with non-small cell lung cancer (NSCLC), and some responses persisted for more than a year. It was also relatively well tolerated; grade ≥3 adverse events occurred in 14% of subjects.

BMS 936559 is a fully human IgG4 monoclonal antibody that targets the PD-1 ligand PD-L1. Phase I results showed that biweekly administration of this drug led to durable responses, especially in subjects with melanoma. Objective response rates ranged from 6% to 17% depending on the cancer type in subjects with advanced-stage NSCLC, melanoma, RCC, or ovarian cancer, with some subjects experiencing responses lasting a year or longer.

MK 3475 is a humanized IgG4 anti-PD-1 monoclonal antibody in phase I development in a five-part study evaluating the dosing, safety, and tolerability of the drug in subjects with progressive, locally advanced, or metastatic carcinoma, melanoma, or NSCLC.

MPDL 3280A is a monoclonal antibody, which also targets PD-L1, undergoing phase I testing in combination with the BRAF inhibitor vemurafenib in subjects with *BRAF* V600- mutant metastatic melanoma and in combination with bevacizumab, which targets vascular endothelial growth factor receptor (VEGFR), with or without chemotherapy in subjects with advanced solid tumors.

AMP 224 is a fusion protein of the extracellular domain of the second PD-1 ligand, PD-L2, and IgG1, which has the potential to block the PD-L2/PD-1 interaction. AMP-224 is currently undergoing phase I testing as monotherapy in subjects with advanced cancer.

Medi 4736 is an anti-PD-L1 antibody in phase I clinical testing in subjects with advanced malignant melanoma, renal cell carcinoma, NSCLC, and colorectal cancer.

CTLA4 (cytotoxic T-lymphocyte-associated protein), is a protein receptor that down regulates the immune system. CTLA4 is found on the surface of T cells, which lead the cellular immune attack on antigens. The T cell attack can be turned on by stimulating the CD28 receptor on the T cell. The T cell attack can be turned off by stimulating the CTLA4 receptor. A first-in-class immunotherapy, ipilimumab (Yervoy), a monoclonal antibody that targets CTLA-4 on the surface of T cells, was for the treatment of melanoma.

Accordingly, the present invention provides a therapeutic agent in combination with an agent that is a checkpoint inhibitor for use in a method of treating a glioma or initiating, enhancing, or prolonging an anti-glioma response in a subject in need thereof comprising administering to the subject a therapeutic agent in combination with an agent that is a checkpoint inhibitor. The checkpoint inhibitor is a monoclonal antibody, a humanized antibody, a fully human antibody or a combination thereof and the checkpoint inhibitor inhibits a checkpoint protein which is PDL1 or or a combination thereof.. In an aspect The therapeutic agent is a glioma lysate-pulsed dendritic cell vaccine. In another aspect, the therapeutic agent is a dendritic cell vaccine, wherein the dendritic cells of the vaccine are partially matured and wherein the partially mature dendritic cells are autologous or allogenic dendritic cells which pick up glioma biomarkers in *situ.*

In a further aspect, the checkpoint inhibitor and the therapeutic are administered simultaneously or sequentially, in either order. In an additional aspect, therapeutic is administered prior to the checkpoint inhibitor and the checkpoint inhibitor is a PD-1 inhibitor.

Use in the claimed treatment is determined by a clinical outcome; an increase, enhancement or prolongation of anti-tumor activity by T cells; an increase in the number of anti-tumor T cells or activated T cells as compared with the number prior to treatment or a combination thereof. In another aspect, clinical outcome is tumor regression; tumor shrinkage; tumor necrosis; anti-tumor response by the immune system; tumor expansion, recurrence or spread or a combination thereof. In an additional aspect, the treatment effect is predicted by presence of T cells, presence of a gene signature indicating T cell inflammation or a combination thereof.

The claimed invention relates to a therapeutic agent in combination with an agent that is a checkpoint inhibitor for use in a method of treating a glioma or initiating, enhancing, or prolonging an anti-glioma response in a subject in need. In some embodiments, the glioma is malignant glioblastoma. In an aspect of the disclosure, the subject has cancer. In an additional aspect of the disclosure which does not form part of the claimed invention, the cancer is any solid tumor or liquid cancers, including urogenital cancers (such as prostate cancer, renal cell cancers, bladder cancers), gynecological cancers (such as ovarian cancers, cervical cancers, endometrial cancers), lung cancer, gastrointestinal cancers (such as non-metastatic or metastatic colorectal cancers, pancreatic cancer, gastric cancer, oesophageal cancers, hepatocellular cancers, cholangiocellular cancers), head and neck cancer (e.g. head and neck squamous cell cancer), malignant mesothelioma, non-metastatic or metastatic breast cancer (e.g. hormone refractory metastatic breast cancer), malignant melanoma, Merkel Cell Carcinoma or bone and soft tissue sarcomas, and haematologic neoplasias, such as multiple myeloma, acute myelogenous leukemia, chronic myelogenous leukemia, myelodysplastic syndrome and acute lymphoblastic leukemia. In a preferred aspect of the disclosure which does not form part of the claimed invention, the disease is non-small cell lung cancer (NSCLC), breast cancer (e.g. hormone refractory metastatic breast cancer), head and neck cancer (e.g. head and neck squamous cell cancer), metastatic colorectal cancers, hormone sensitive or hormone refractory prostate cancer, colorectal cancer, ovarian cancer, hepatocellular cancer, renal cell cancer, soft tissue sarcoma, or small cell lung cancer.

In a further aspect, the use in the method further comprises administering a chemotherapeutic agent, targeted therapy or radiation to the subject either prior to, simultaneously with, or after treatment with the combination therapy. In an additional aspect, the tumor may be resected prior to the administration of the therapeutic and checkpoint inhibitor.

The agent that is a checkpoint inhibitor of the invention is selected from the group consisting of a monoclonal antibody, a humanized antibody and a fully human antibody or combination thereof and inhibits a checkpoint protein selected from the group consisting of PD-1 and PDL1 or a combination thereof. Checkpoint inhibitors of the disclosure include any agent that blocks or inhibits in a statistically significant manner, the inhibitory pathways of the immune system. Such inhibitors of the disclosure may include small molecule inhibitors or may include antibodies, or antigen binding fragments thereof, that bind to and block or inhibit immune checkpoint receptors or antibodies that bind to and block or inhibit immune checkpoint receptor ligands. Illustrative checkpoint molecules that may be targeted for blocking or inhibition include, but are not limited to, CTLA-4, PDL1, PDL2, PD1, B7-H3, B7-H4, BTLA, HVEM, GAL9, LAG3, TIM3, VISTA, KIR, 2B4 (belongs to the CD2 family of molecules and is expressed on all NK, γδ, and memory CD8⁺ (αβ) T cells), CD160 (also referred to as BY55), CGEN-15049, CHK 1 and CHK2 kinases, A2aR and various B-7 family ligands. B7 family ligands include, but are not limited to, B7-1, B7-2, B7-DC, B7-H1, B7-H2, B7-H3, B7-H4, B7-H5, B7-H6 and B7-H7. Checkpoint inhibitors include antibodies, or antigen binding fragments thereof, other binding proteins, biologic therapeutics or small molecules, that bind to and block or inhibit the activity of one or more of CTLA-4, PDL1, PDL2, PD1, BTLA, HVEM, TIM3, GAL9, LAG3, VISTA, KIR, 2B4, CD160 and CGEN-15049. Illustrative immune checkpoint inhibitors include Tremelimumab (CTLA-4 blocking antibody), anti-OX40, PD-L1 monoclonal Antibody (Anti-B7-H1; MEDI4736), MK-3475 (PD-1 blocker), Nivolumab (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor). Checkpoint protein ligands include, but are not limited to PD-L1, PD-L2, B7-H3, B7-H4, CD28, CD86 and TIM-3. Checkpoint inhibitors that inhibit a checkpoint protein other than PD-1, PDL1 or a combination thereof do not form part of the claimed invention.

The present invention uses a specific class of checkpoint inhibitor are drugs that block the interaction between immune checkpoint receptor programmed cell death protein 1 (PD-1) and its ligand PDL-1. *See* A. Mullard, "New checkpoint inhibitors ride the immunotherapy tsunami," Nature Reviews: Drug Discovery (2013), 12:489-492. PD-1 is expressed on and regulates the activity of T-cells. Specifically, when PD-1 is unbound to PDL-1, the T-cells can engage and kill target cells. However, when PD-1 is bound to PDL-1 it causes the T-cells to cease engaging and killing target cells. Furthermore, unlike other checkpoints, PD-1 acts proximately such the PDLs are overexpresseed direcly on cancer cells which leads to increased binding to the PD-1 expressing T-cells.

The invention provides checkpoint inhibitors which are antibodies that can act as agonists of PD-1, thereby modulating immune responses regulated by PD-1. In one aspect of the disclosure, the anti-PD-1 antibodies can be antigen-binding fragments. Anti-PD-1 antibodies disclosed herein are able to bind to human PD-1 and agonize the activity of PD-1, thereby inhibiting the function of immune cells expressing PD-1.

In one aspect, which does not form part of the claimed invention, the disclosure covers the use of a specific class of checkpoint inhibitor are drugs that inhibit CTLA-4. Suitable anti-CTLA4 antagonist agents for use in the methods of the disclosure, include, without limitation, anti-CTLA4 antibodies, human anti-CTLA4 antibodies, mouse anti-CTLA4 antibodies, mammalian anti-CTLA4 antibodies, humanized anti-CTLA4 antibodies, monoclonal anti-CTLA4 antibodies, polyclonal anti-CTLA4 antibodies, chimeric anti-CTLA4 antibodies, MDX-010 (ipilimumab), tremelimumab, anti-CD28 antibodies, anti-CTLA4 adnectins, anti-CTLA4 domain antibodies, single chain anti-CTLA4 fragments, heavy chain anti-CTLA4 fragments, light chain anti-CTLA4 fragments, inhibitors of CTLA4 that agonize the co-stimulatory pathway, the antibodies disclosed in PCT Publication No. WO 2001/014424, the antibodies disclosed in PCT Publication No. WO 2004/035607, the antibodies disclosed in U.S. Publication No. 2005/0201994, and the antibodies disclosed in granted European Patent No. EP 1212422 B1. Additional CTLA-4 antibodies are described in U.S. Pat. Nos. 5,811,097, 5,855,887, 6,051,227, and 6,984,720; in PCT Publication Nos. WO 01/14424 and WO 00/37504; and in U.S. Publication Nos. 2002/0039581 and 2002/086014. Other anti-CTLA-4 antibodies that can be used in a method of the present disclosure include, for example, those disclosed in: WO 98/42752; U.S. Pat. Nos. 6,682,736 and 6,207,156; Hurwitz et al., Proc. Natl. Acad. Sci. USA, 95(17):10067-10071 (1998); Camacho et al., J. Clin. Oncology, 22(145):Abstract No. 2505 (2004) (antibody CP-675206); Mokyr et al., Cancer Res., 58:5301-5304 (1998), and U.S. Pat. Nos. 5,977,318, 6,682,736, 7,109,003, and 7,132,281.

Additional anti-CTLA4 antagonists include, but are not limited to, the following: any inhibitor that is capable of disrupting the ability of CD28 antigen to bind to its cognate ligand, to inhibit the ability of CTLA4 to bind to its cognate ligand, to augment T cell responses via the co-stimulatory pathway, to disrupt the ability of B7 to bind to CD28 and/or CTLA4, to disrupt the ability of B7 to activate the co-stimulatory pathway, to disrupt the ability of CD80 to bind to CD28 and/or CTLA4, to disrupt the ability of CD80 to activate the co-stimulatory pathway, to disrupt the ability of CD86 to bind to CD28 and/or CTLA4, to disrupt the ability of CD86 to activate the co-stimulatory pathway, and to disrupt the co-stimulatory pathway, in general from being activated. This necessarily includes small molecule inhibitors of CD28, CD80, CD86, CTLA4, among other members of the co-stimulatory pathway; antibodies directed to CD28, CD80, CD86, CTLA4, among other members of the co-stimulatory pathway; antisense molecules directed against CD28, CD80, CD86, CTLA4, among other members of the co-stimulatory pathway; adnectins directed against CD28, CD80, CD86, CTLA4, among other members of the co-stimulatory pathway, RNAi inhibitors (both single and double stranded) of CD28, CD80, CD86, CTLA4, among other members of the co-stimulatory pathway, among other anti-CTLA4 antagonists.

In one specific aspect which does not form part of the claimed invention, the disclosure covers the use of a specific class of checkpoint inhibitor are drugs that inhibit TIM-3. Blocking the activation of TIM-3 by a ligand, results in an increase in Th1 cell activation. Furthermore, TIM-3 has been identified as an important inhibitory receptor expressed by exhausted CD8+ T cells. TIM-3 has also been reported as a key regulator of nucleic acid mediated antitumor immunity. In one example, TIM-3 has been shown to be upregulated on tumor-associated dendritic cells (TADCs).

In one specific aspect, which does not form part of the claimed invention, the disclosure is directed to the use of immunostimulatory agents, T cell growth factors and interleukins. Immunostimulatory agents are substances (drugs and nutrients) that stimulate the immune system by inducing activation or increasing activity of any of its components. Immunostimulants include bacterial vaccines, colony stimulating factors, interferons, interleukins, other immunostimulants, therapeutic vaccines, vaccine combinations and viral vaccines.

T cell growth factors are proteins which stimulate the proliferation of T cells. Examples of T cell growth factors include Il-2, IL-7, IL-15, IL-17, IL21 and IL-33.

Interleukins are a group of cytokines that were first seen to be expressed by white blood cells. The function of the immune system depends in a large part on interleukins, and rare deficiencies of a number of them have been described, all featuring autoimmune diseases or immune deficiency. The majority of interleukins are synthesized by helper CD4 T lymphocytes, as well as through monocytes, macrophages, and endothelial cells. They promote the development and differentiation of T and B lymphocytes, and hematopoietic cells. Examples of interleukins include IL-1, IL-2, IL-3, IL-4, IL-5, IL-6, IL-7, IL-8, IL-9, IL-10, IL-11, IL-12, IL-13, IL-14, IL-15 and IL-17.

IL-15 is a cytokine that binds to and signals through a complex composed of IL-2/IL-15 receptor beta chain (CD122) and the common gamma chain (gamma-C, CD132). IL-15 is secreted by mononuclear phagocytes following infection by viruses. This cytokine induces cell proliferation of natural killer cells; cells of the innate immune system whose principal role is to kill virally infected cells.

IL-7 is a hematopoietic growth factor secreted by stromal cells in the bone marrow and thymus. IL-7 stimulates the differentiation of multipotent and pluripotent hematopoietic stem cells into lymphoid progenitor cells (as opposed to myeloid progenitor cells where differentiation is stimulated by IL-3). It also stimulates proliferation of all cells in the lymphoid lineage (B cells, T cells and NK cells). It is important for proliferation during certain stages of B-cell maturation, T and NK cell survival, development and homeostasis. IL-7 may be glycosylated.

In certain methods or compositions described herein, an additional third, fourth, fifth, sixth, seventh, eighth, ninth, or tenth agent. In certain embodiments, the additional third, fourth, fifth, sixth, seventh, eighth, ninth, or tenth agent is a chemotherapeutic agent, a cytokine therapy, an interferon therapy (e.g., INF-α), an interlukin therapy (*e.g.,* IL-2, IL-7, or IL-11), a colony-stimulting factor therapy (e.g., G-CSF), an antibody therapy, a viral therapy, gene therapy or a combination thereof. In certain embodiments, the additional third, fourth, fifth, sixth, seventh, eighth, ninth, or tenth agent can be used prior to, concurrent with, or after treatment with any of the methods or compositions described herein.

Examples of cancer therapeutic agents or chemotherapeutic agents include alkylating agents such as thiotepa and cyclophosphamide (CYTOXAN); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as aclacinomysins, actinomycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK.RTM.; razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{™}, Bristol-Myers Squibb Oncology, Princeton, N.J.) and doxetaxel (TAXOTERE^{™}, Rhne-Poulenc Rorer, Antony, France); chlorambucil; gemcitabine; 6-thioguanine; mercaptopurine; methotrexate; platinum analogs such as cisplatin and carboplatin; vinblastine; trastuzumab, docetaxel, platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylomithine (DMFO); retinoic acid derivatives such as Targretin ^{™} (bexarotene), Panretin^{™} (alitretinoin); ONTAKT^{™} (denileukin diftitox); esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above. Further cancer therapeutic agents include sorafenib and other protein kinase inhibitors such as afatinib, axitinib, bevacizumab, cetuximab, crizotinib, dasatinib, erlotinib, fostamatinib, gefitinib, imatinib, lapatinib, lenvatinib, mubritinib, nilotinib, panitumumab, pazopanib, pegaptanib, ranibizumab, ruxolitinib, trastuzumab, vandetanib, vemurafenib, and sunitinib; sirolimus (rapamycin), everolimus and other mTOR inhibitors.

Examples of additional chemotherapeutic agents include topoisomerase I inhibitors (*e.g.,* irinotecan, topotecan, camptothecin and analogs or metabolites thereof, and doxorubicin); topoisomerase II inhibitors (*e.g.,* etoposide, teniposide, and daunorubicin); alkylating agents (*e.g.,* melphalan, chlorambucil, busulfan, thiotepa, ifosfamide, carmustine, lomustine, semustine, streptozocin, decarbazine, methotrexate, mitomycin C, and cyclophosphamide); DNA intercalators (*e.g.,* cisplatin, oxaliplatin, and carboplatin); DNA intercalators and free radical generators such as bleomycin; and nucleoside mimetics (*e.g.,* 5-fluorouracil, capecitibine, gemcitabine, fludarabine, cytarabine, mercaptopurine, thioguanine, pentostatin, and hydroxyurea). Moreover, exemplary chemotherapeutic agents that disrupt cell replication include: paclitaxel, docetaxel, and related analogs; vincristine, vinblastin, and related analogs; thalidomide, lenalidomide, and related analogs (*e.g.,* CC-5013 and CC-4047); protein tyrosine kinase inhibitors (*e.g.,* imatinib mesylate and gefitinib); proteasome inhibitors (*e.g.,* bortezomib); NF-κB inhibitors, including inhibitors of IxB kinase; antibodies which bind to proteins overexpressed in cancers and other inhibitors of proteins or enzymes known to be upregulated, over-expressed or activated in cancers, the inhibition of which downregulates cell replication.

The present invention utilizes a glioma lysate-pulsed dendritic cell vaccine, or a dendritic cell vaccine, wherein the dendritic cells of the vaccine are partially matured and wherein the partially mature dendritic cells are autologous or allogenic dendritic cells which pick up glioma biomarkers in situ. In one specific aspect, the present disclosure is directed to the use of therapeutic vaccines, including cancer vaccines and dendritic cell vaccines. The goal of cancer vaccines is to get the immune system to mount an attack against cancer cells in the body. In other words, cancer vaccines work by priming the immune system to attack cancer cells in the body. Accordingly, instead of preventing disease, cancer vaccines are meant to get the immune system to attack a disease that already exists. A cancer vaccine uses cancer cells, parts of cells, or pure antigens to increase the immune response against cancer cells that are already in the body.

Cancer vaccines, unlike traditional immune boosting therapies, don't just improve the immune system in general, they cause the immune system to attack cells with one or more specific antigens and create an "attack memory" in the immune system. This "attack memory" allows the immune system to continue attacking the cancer cells to prevent cancers from progressing and/or returning once put into remission.

Cancer vaccines can be made from actual cancer cells that have been removed from a subject. Once removed, the cancer cells are modified in the lab, usually with radiation, so they cannot form more tumors. In the lab, it is also common for scientists to further modify the cancer cells. For example, the cancer cells are often modified by adding chemicals or new genes, to make the cells more likely to be seen as foreign by the immune system. The modified cells are then injected back into the subject. The immune system is able to recognize the antigens on these cells and through natural physiological processes seeks out and attacks/kills cells that express the intended antigen.

Most vaccines are autologous. Autologous vaccines are made from killed (*e.g.,* treated with radiation or chemicals to render the cancer cells incapable of replication) taken from the same person in whom they will later be used. In other words, cells are taken from a subject, modified, and then injected back into the same subject.

Other vaccines are allogeneic. Allogenic vaccines are made from cells taken from one subject and then injected into a second, different, subject. While allogenic vaccines are easier to make than autologous vaccines, there may be benefits to using autologous vaccines to avoid introduction of foreign cells into a subject.

There are multiple types of cancer vaccines. Non-limiting examples of cancer vaccines include tumor cell vaccines, antigen vaccines, dendritic cell vaccines, DNA vaccines, and vector based vaccines.

Antigen vaccines boost the immune system by using one or more antigens, in contrast to whole tumor cells that contain many thousands of antigens. These antigens are generally peptides. Antigen vaccines may be specific for a certain type of cancer because each tumor type may be identified by specific antigen profiles. In order to maximize the efficacy of these vaccines, it may be beneficial to combine multiple antigens in the vaccine depending on the antigen profile of a specific cancer.

Dendritic cell vaccines are often autologous vaccines, and must often be made individually for each subject. The process used to create them is complex and expensive. Doctors remove some immune cells from the blood and expose them in the lab to cancer cells or cancer antigens, as well as to other chemicals that turn them into dendritic cells and help them grow. The dendritic cells are then injected back into the subject, where they should provoke an immune response to cancer cells in the body. A non-limiting example of a dendritic vaccine is Sipuleucel-T (described below).

DNA vaccines are a third non-limiting cancer cell vaccine. One limitation of traditional cancer cell vaccines is that when tumor cells or antigens are injected into the body as a vaccine, they may cause the desired immune response at first, but they may become less effective over time. This is because the immune system recognizes them as foreign and quickly destroys them. Without any further stimulation, the immune system often returns to its normal (pre-vaccine) state of activity. One way of promoting the continued immune response is using DNA vaccines.

DNA is the substance in cells that contains the genetic code for the proteins that cells make. Vectors can be engineered to contain specific DNAs that can be injected into a subject which leads to the DNA being taken up by cells. Once the cells take up the DNA, the DNA will program the cells to make specific antigens, which can then provoke the desired immune response.

The fourth non-limiting cancer vaccine is a vector composition. Vector vaccines can be used to administer the DNA of DNA vaccines. Vectors are special viruses, bacteria, yeast cells, or other structures that can be used to get antigens or DNA into the cells of a subject. Vectors are particularly useful because they may be used to deliver more than one cancer antigen at a time, which may make a subject's immune system more likely to mount a response. Vectors are also particularly useful because they can trigger an immune response on its own (without any additional DNA or antigen) which will yield a stronger immune response when combined with a DNA and/or antigen.

Dendritic cells can be administered directly into a tumor, into the tumor bed subsequent to surgical removal or resection of the tumor, peri-tumorily, into a draining lymph node in direct contact with the tumor, into a blood vessel or lymph duct leading into, or feeding a tumor or organ afflicted by the tumor, e.g., the portal vein or a pulmonary vein or artery, and the like. The administration of partially mature dendritic cells can be either simultaneous with or subsequent to other treatments for the tumor, such as chemotherapy or radiation therapy. Further, partially mature dendritic cells can be co-administered with another agent, which agent acts as an adjuvant to the maturation of the dendritic cell and/or the processing of antigen within the tumor or region near or adjacent to the tumor. In addition, the dendritic cells can also be formulated or compounded into a slow release matrix for implantation into a region in or around the tumor or tumor bed such that cells are slowly released into the tumor, or tumor bed, for contact with the tumor antigens.

Partially mature dendritic cells can also be administered by any means appropriate for the formulation and mode of administration. For example, the cells can be combined with a pharmaceutically acceptable carrier and administered with a syringe, a catheter, a cannula, and the like. As above, the cells can be formulated in a slow release matrix. When administered in this fashion, the formulation can be administered by a means appropriate for the matrix used. Other methods and modes of administration applicable to the present invention are well known to the skilled artisan.

Dendritic cell compositions can be used by themselves in the treatment of an individual. In addition, the compositions can be used in combination with any other method to treat a tumor. For example, use in the methods of the present invention can be used in combination with surgical resection of a tumor, chemotherapy (cytotoxic drugs, apoptotic agents, antibodies, and the like), radiation therapy, cryotherapy, brachytherapy, immune therapy (administration of antigen specific mature activated dendritic cells, NK cells, antibodies specific for tumor antigens, etc.), and the like. Any and all of these methods can also be used in any combination. Combination treatments can be concurrent or sequential and can be administered in any order as determined by the treating physician.

One example of a commercially available cancer vaccine is Sipuleucel-T or Provenge^{®}. It is a cancer cell vaccine that is used to treat advanced prostate cancer that is not treatable by traditional chemotherapeutic or hormone therapies. For this vaccine, a subject's own immune cells are isolated from the subject and the immune cells are then exposed to chemicals to convert them into dendritic cells. The dendritic cells are exposed to prostatic acid phosphatase (PAP) which, when reintroduced into the subject, produces an immune response against prostate cancer. Of particular importance, once the modified cells are reintroduced into the subject, the subject's immune system creates an "attack memory" and transforms other immune cells within the subject into cancer attacking cells.

One example of a dendritic cell vaccine is DCVax. DCVax is a platform technology that uses activated dendritic cells (the master cells of the immune system), and is designed to reinvigorate and educate the immune system to attack cancers. DCVax uses many active agents to hit many targets on the cancer (Liau, LM et al. Journal of Neurosurgery 90: 1115-1124, 1999; Prins RM et al. J Immunother. 2013 Feb; 36(2):152-7).

There are three key aspects of dendritic cell vaccines, including, DCVax, that make the vaccines effective in treating cancer: (1) dendritic cell vaccines are designed to mobilize the entire immune system, not just one among the many different categories of immune agents in that overall system. As described above, DCVax is comprised of activated, educated dendritic cells, and dendritic cells are the master cells of the immune system, that mobilize or help the entire immune system. Full immune system involves many types of antibodies, and also many other kinds of agents besides antibodies. Dendritic cells mobilize all of these different categories of agents, comprising the whole immune system "army," in combination with each other and in their natural relationships to each other. (2) dendritic cell vaccines are designed to target not just one but the full set of biomarkers on the subject's tumor, which may make it more difficult for tumors to mutate and metastasize. (3) dendritic cell vaccines are personalized, and targets the particular biomarkers expressed on that subject's tumor.

To make a dendritic cell vaccine for a subject, the subject's immune cells are obtained through a blood draw. For systemic administration, the monocytes are differentiated into dendritic cells, matured, activated and loaded with biomarkers from the subject's own tumor tissue. The loading of biomarkers into the dendritic cells "educates" the cells about what the immune system needs to attack. The activated, educated dendritic cells are then isolated with very high purity and are administered to the subject through a simple intradermal injection in the upper arm. The dendritic cells then convey the tumor biomarker information to the rest of the immune system agents (T cells, B cells and others) which then target cells with these biomarkers.

For intratumoral administration, the monocytes are differentiated into dendritic cells and partially matured. The cells are then administered by injection directly into the tumors. After injection into the tumors, dendritic cells pick up the tumor biomarkers in situ and convey the tumor biomarker information to the rest of the immune system agents (T cells, B cells and others) which then target cells with these biomarkers.

Importantly, each activated, educated dendritic cell in the vaccine has a large multiplier effect, mobilizing hundreds of T cells and other immune cells. As a result, small doses of such dendritic cells can mobilize large and sustained immune responses.

In one embodiment, dendritic cell compositions can be used as a first treatment, or a primer, for a second checkpoint inhibitor treatment. By administering the dendritic cell compositions before the checkpoint inhibitor(s), the immune system (specifically T-cells) become activated which allows an enhanced response to checkpoint inhibitors.

In one embodiment, the checkpoint inhibitor is administered first to 'unblock' the initiation of an immune response, followed a cancer vaccine therapy. For example, a checkpoint inhibitor is administered to a subject followed by a dendritic cell composition.

In a specific embodiment, the dendritic cells and the recipient subject have the same MHC (HLA) haplotype. Methods of determining the HLA haplotype of a subject are known in the art. In a related embodiment, the partially mature dendritic cells are allogenic to the recipient subject. The allogenic cells are typically matched for at least one MHC allele (e.g., sharing at least one but not all MHC alleles). In a less typical embodiment, the dendritic cells and the recipient subject are all allogeneic with respect to each other, but all have at least one MHC allele in common.

Administration includes administering one or more cycles or doses of a checkpoint inhibitor prior to, simultaneously with or following administration of a therapeutic, e.g., interleukins such as IL-7 or IL-15 or a vaccine, such a dendritic vaccine or vice versa. One of skill in the art can determine which therapeutic regimen is appropriate on a subject by subject basis, depending on their cancer and their immune status (e.g., T-cell, B cell or NK cell activity and/or numbers).

The combination of a check point inhibitor and a therapeutic can be more effective in treating cancer in some subjects and/or can initiate, enable, increase, enhance or prolong the activity and/or number of immune cells (including T cells, B cells, NK cells and/or others) or convey a medically beneficial response by a tumor (including regression, necrosis or elimination thereof).

In certain aspects, an immune response is induced prior to the administration of a checkpoint inhibitor to initiate or enable an anti-tumor immune response using autologous or allogeneic dendritic cells loaded with autologous or allogeneic (*e.g.,* from cell lines) tumor antigens, followed by administration of checkpoint inhibitor(s).

In certain aspects, an immune response is induced prior to the administration of a checkpoint inhibitor to enhance a pre-existing anti-tumor immune response using autologous or allogeneic dendritic cells loaded with autologous or allogeneic (*e.g.,* from cell lines) tumor antigens, followed by administration of checkpoint inhibitor(s).

In certain aspects, an immune response is induced or enhanced prior to the administration of a checkpoint inhibitor to enhance a pre-existing anti-tumor immune response using autologous or allogeneic T cells specific for autologous tumor antigens, followed by administration of checkpoint inhibitor(s).

In certain aspects, an immune response is induced prior to the administration of a checkpoint inhibitor to initiate or enable an anti-tumor immune response using autologous or allogeneic dendritic cells administered directly into or peripherally to a tumor for in vivo antigen loading, followed by administration of checkpoint inhibitor(s).

In certain aspects, an immune response is induced prior to the administration of a checkpoint inhibitor to enhance a pre-existing anti-tumor immune response using autologous or allogeneic dendritic cells administered directly into or peripherally to a tumor for in vivo antigen loading, followed by administration of checkpoint inhibitor(s).

In certain aspects, an immune response is induced prior to the administration of a checkpoint inhibitor to initiate an anti-tumor immune response using allogeneic dendritic cells loaded with autologous tumor antigens, followed by administration of checkpoint inhibitor(s).

In certain aspects, an immune response is induced prior to the administration of a checkpoint inhibitor to enhance a pre-existing anti-tumor immune response using allogeneic dendritic cells loaded with autologous tumor antigens, followed by administration of checkpoint inhibitor(s).

In certain aspects, an immune response is induced prior to the administration of a checkpoint inhibitor to initiate an anti-tumor immune response using allogeneic dendritic cells administered directly into or peripherally to a tumor for in vivo antigen loading, followed by administration of checkpoint inhibitor(s).

In certain aspects, an immune response is induced prior to the administration of a checkpoint inhibitor to enhance a pre-existing anti-tumor immune response using allogeneic dendritic cells administered directly into or peripherally to a tumor for in vivo antigen loading, followed by administration of checkpoint inhibitor(s).

In certain aspects which do not form part of the claimed invention, an immune response is induced prior to the administration of a checkpoint inhibitor to initiate an anti-tumor immune response using autologous anti-tumor T cells which are expanded and or activated *ex vivo,* followed by administration of checkpoint inhibitor(s).

In certain aspects which do not form part of the claimed invention, an immune response is induced prior to the administration of a checkpoint inhibitor to enhance a pre-existing anti-tumor immune response using anti-tumor T-cells which are expanded and or activated *ex vivo,* followed by administration of checkpoint inhibitor(s).

In certain aspects of the disclosure, the therapeutic cancer vaccine or adoptive T cell therapy is administered chronologically before the checkpoint inhibitor. In certain aspects of the disclosure, the therapeutic cancer vaccine or adoptive T cell therapy is administered 1 hour, 2 hours, 3 hours, 4 hours, 5 hours, 6 hours, 7 hours, 8 hours, 9 hours, 10 hours, 11 hours, 12 hours, 1 day, 2 days, 3 days, 4 days, 5 days, 6 days, 7 days, 8 days, 9, days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 1 month, or any combination thereof, before the checkpoint inhibitor is administered.

In certain aspects of the present disclosure, the therapeutic cancer vaccine or adoptive T cell therapy is administered chronologically at the same time as the checkpoint inhibitor.

In certain aspects of the present disclosure, the therapeutic cancer vaccine or adoptive T cell therapy is administered chronologically after the checkpoint inhibitor. In certain embodiments, the checkpoint inhibitor is administered 1 hour, 2 hours, 3 hours, 4 hours, 5 hours, 6 hours, 7 hours, 8 hours, 9 hours, 10 hours, 11 hours, 12 hours, 1 day, 2 days, 3 days, 4 days, 5 days, 6 days, 7 days, 8 days, 9, days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 1 month, or any combination thereof, before the therapeutic cancer vaccine or adoptive T cell therapy is administered.

In all of the aspects described above, the use in methods of inducing, initiating, or enhancing an anti-tumor response or an immune response can be accomplished by administering the checkpoint inhibitor first and the second agent to inducing, initiating, or enhancing an anti-tumor response or an immune response at a time point thereafter.

In another embodiment, the present invention provides for use in a method of enhancing or prolonging the effects of a checkpoint inhibitor, or enabling a subject to respond to a checkpoint inhibitor, or enabling the toxicity or the dose of a checkpoint inhibitor to be reduced, comprising administering to a subject in need thereof a therapeutic in combination with a checkpoint inhibitor wherein the subject has cancer.

In a further aspect, which does not form part of the claimed invention, the disclosure provides for a pharmaceutical composition comprising a checkpoint inhibitor in combination with a biologic therapeutic. In one aspect, the biologic therapeutic is a vaccine.

An immune response is induced, initiated, or enhanced using a dendritic cell vaccine as recited in the claims. In specific aspects which do not form part of the claimed invention, an immune response can be induced, initiated, or enhanced using anti-tumor T-cells. In specific aspects which do not form part of the claimed invention, an immune response can be induced, initiated, or enhanced through the induction of cytotoxic T-cells.

In specific embodiments, the therapeutic agent in combination with an agent that is a checkpoint inhibitor are used to treat a glioma or initiating, enhancing, or prolonging an anti-glioma response in a subject in need thereof. Specifically, the therapeutic agent in combination with an agent that is a checkpoint inhibitor can be used to decrease the size of a solid tumor or decrease the number of cancer cells of the cancer. The therapeutic agent in combination with an agent that is a checkpoint inhibitor can be used to slow the rate of cancer cell growth. The therapeutic agent in combination with an agent that is a checkpoint inhibitor can be used to stop the rate of cancer cell growth.

The use of the therapeutic, checkpoint inhibitor, biologic therapeutic or pharmaceutical composition as disclosed herein can be administered to an individual by various routes including, for example, orally or parenterally, such as intravenously, intramuscularly, subcutaneously, intraorbitally, intracapsularly, intraperitoneally, intrarectally, intracisternally, intratumorally, intravasally, intradermally or by passive or facilitated absorption through the skin using, for example, a skin patch or transdermal iontophoresis, respectively. The use of the therapeutic, checkpoint inhibitor, biologic therapeutic or pharmaceutical composition also can be administered to the site of a pathologic condition, for example, intravenously or intra-arterially into a blood vessel supplying a tumor.

The total amount of an agent to be administered in practicing use in a method of the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary. In general, the formulation of the composition and the routes and frequency of administration are determined, initially, using Phase I and Phase II clinical trials.

In certain embodiments, the checkpoint inhibitor is administered in 0.01mg/kg, 0.05mg/kg, 0.1mg/kg, 0.2mg/kg, 0.3mg/kg, 0.5mg/kg, 0.7mg/kg, 1mg/kg, 2mg/kg, 3mg/kg, 4mg/kg, 5mg/kg, 6mg/kg, 7mg/kg, 8mg/kg, 9mg/kg, 10mg/kg, or any combination thereof doses. In certain embodiments the checkpoint inhibitor is administered once a week, twice a week, three times a week, once every two weeks, or once every month. In certain embodiments, the checkpoint inhibitor is administered as a single dose, in two doses, in three doses, in four doses, in five doses, or in 6 or more doses.

In certain aspects of the disclosure which do not form part of the present invention, the methods and compositions described herein are packaged in the form of a kit. In certain aspects, the instructions for performing the methods and using the compositions are included in the kits.

In other aspects of the disclosure which do not form part of the present invention, an article of manufacture containing materials useful for the treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). The active agent in the composition is the antibody. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

As shown in the Figures and Examples, the PD-1/PD-L1 negative co-stimulatory axis in the glioma microenvironment is characterized and noted increased tumor-infiltrating regulatory cell populations (Tregs, iAPCs) associated with lysate-pulsed dendritic cell vaccination. Blockade of this mechanism with adjuvant anti-PD-1 Ab effects cytotoxic T cell activation and trafficking to tumor and promotes a non-inhibitory tumor environment. Combinatorial DC vaccination and anti-PD-1 Ab therapy promotes significant long-term survival in murine glioma models. These mechanisms have been delineated and provide a practical clinical imaging correlate utilizing MR and novel PET imaging probes to non-invasively characterize immune function *in vivo.*

Specifically, the Figures show that in mice bearing well-established intracranial gliomas, tumor lysate-pulsed DC vaccination still results in significant infiltration of activated T lymphocytes, but without any clinical benefit. Further, a population of inhibitory myeloid cells accumulates within intracranial GL261 gliomas, and this population significantly increases after DC vaccination. Adjuvant treatment of PD-1 blocking antibody together with tumor lysate-pulsed DC vaccination prevents the enhanced accumulation of inhibitory myeloid cells within intracranial GL261 gliomas. This results in a significant increase in the intratumoral accumulation of activated T lymphocytes, dramatic extension of survival, and the generation of immune memory to the tumor. Further, overnight pulsing of tumor lysate onto DC activates these cells and results in reduced expression of PD-L1, suggesting that activation and/or maturation will reduce the inhibitory immune function of because inhibitory myeloid cells are a bone marrow-derived precursor population of DC and macrophages.

The following examples are provided to further illustrate the embodiments of the present invention, but are not intended to limit the scope of the invention which is defined by the claims. While they are typical of those that might be used, other procedures, methodologies, or techniques known to those skilled in the art may alternatively be used. Any examples falling outside the scope of the claims are provided for comparative purposes.

### EXAMPLE 1

### Glioma microenvironment negates anti-tumor immune response promoted by DC vaccination

There may be limited endogenous immune response to tumor in C57BL/6 mice intracranially implanted with GL261 murine glioma (Figure 1A). Mice were intracranially implanted with GL261 murine glioma and then administered PBS (1x) or lysate-pulsed DC vaccine subcutaneously on days 3 and 13 post-tumor implant. On day 16, 72h after the second treatment, mice were euthanized and spleen, lymph, and brain hemispheres harvested for processing. Vaccination with lysate-pulsed dendritic cells promotes significant tumor infiltration of CD3+ lymphocytes, of which a majority are activated CD8+ CD25+ lymphocytes. To determine the overall physiologic effect of these cell populations, two groups of GL261 glioma-bearing mice (control and DC vaccine-treated) were maintained and monitored survival. No survival benefit between the two groups was noted when the DC vaccination was given to large intracranial tumors (Figure 1C).

### EXAMPLE 2

### PD-1/PD-L1 T cell-glioma interaction promotes an anti-inflammatory tumor microenvironment

To evaluate the effect of PD-1/PD-L1 tumor-T cell interaction, gp-100-specific T cells from a Pmel-1 TCR transgenic mouse were co-cultured with GL261-gp100 murine glioma cells in the presence of anti-PD-1 mAb. Supernatant collected 24h later was processed and analyzed with the mouse 32-plex cytokine/chemokine Luminex assay. Pro-inflammatory cytokines IFNγ and TNFα showed significant increase, while anti-inflammatory signaling (IL-10 and IL-4) decreased with PD-1 inhibition. Cytotoxicity was evaluated using the xCELLigence system, which offers a real-time, impedance-based readout of tumor killing by T cells. Inhibition of PD-1 effectively supported greater percent kill of tumor cells at the 10h time point.

### EXAMPLE 3

### Inhibition of the PD-1/PD-L1 negative costimulatory axis in glioma-bearing mice promotes anti-tumoral response

It was hypothesized that anti-tumor response promoted by the vaccination treatment is mitigated by PD-1/PD-L1 signaling in the tumor microenvironment. There was a significant inhibitory myeloid population (Ly6C+) expressing PD-L1 present in tumors harvested from DC vaccine-treated mice that was not present in control mice. To evaluate this population, two additional therapies to control and DC-vaccine treatment were examined: anti-PD-1 mAb-treated and combination DC vaccine with anti-PD-1 mAb-treated groups. Spleen, lymph, and brain hemispheres harvested for processing on day 16 post-implant (72h after second treatment). It was noted significant activated cytotoxic TILs and lymph nodes of DC vaccine/anti-PD-1 treated mice. While the inhibitory myeloid population persisted in the DC vaccine/anti-PD-1 treated mice, it was significantly reduced when compared to DC vaccine treatment alone. In anti-PD-1 mice, it was not significantly detectable. To evaluate therapeutic benefit, mice were implanted, treated, and monitored for survival. Combination DC vaccine/anti-PD-1 treatment showed significant survival benefit over other groups.

The inhibitory myeloid population was depleted utilizing Ly6C depleting Ab or the clinically-relevant CSF1r inhibitory drug PLX3397. Depletion of these cells in GL261-bearing mice entirely recovered survival benefit observed in mice treated with DC vaccine/anti-PD-1. Spleen, lymph, and brain hemispheres harvested for processing on day 16 post-implant (72h after second treatment). The absence of these Ly6C+ PD-L1+ cells in the tumor microenvironment was confirmed and noted a significant tumor-infiltrating population of CD3+ CD8+ CD25+ activated lymphocytes. Depletion of CD8+ cells in mice treated with DC vaccine/anti-PD-1 abolished all therapeutic benefit associated with the treatment (Figure 4c). Tissue harvests confirmed the absence of activated lymphocytes both systemically and at the tumor site.

### EXAMPLE 4

### Novel use of PET and MR imaging modalities allows for non-invasive evaluation of the DC vaccine/anti-PD-1 therapy

It was hypothesized that the therapeutic progress of the DC vaccine/anti-PD-1 therapy could be monitored utilizing MRI (magnetic resonance imaging) and PET (positron emission topography) imaging techniques. Mice intracranially implanted with GL261 were separated into four treatment groups (no treatment, DC vaccine, anti-PD-1 mAb, and DC vaccine/anti-PD-1) and imaged with either [18F]-Z-FAC or [18F]-D-FAC PET probes to image activated lymphocyte trafficking to tumor. On day 21 post-implant, mice were imaged using a Bruker 7T MR scanner (UCLA) to obtain pre- and post-contrast T1-weighted images, T2 maps, and dynamic contrast-enhancing (DCE) and dynamic susceptibility contrast (DSC) perfusion data. Following imaging, mice were euthanized and brain tissue harvested for sectioning and IHC. PET and post-contrast T1-weighted MR data was co-registered to delineate positive MR tumor contrast against positive PET probe signal. DC vaccine-treated and DC vaccine/anti-PD-1 treated groups showed decreased tumor burden and increased immune infiltration when compared to other treatment groups. Positive PET and MR signal correlated to CD3+ and Ki-67+ IHC staining, respectively, on equivalent anatomic brain tissue sections.

### EXAMPLE 5

### Materials & Methods

*Cell lines.* The murine glioma cell lines, GL261 and GL261-gp100 were maintained in complete DMEM (Mediatech, Inc. Herndon, VA) (supplemented with 10% FBS (Gemini Bio-Products, West Sacramento, CA), 1% (v/v) penicillin and streptomycin (Mediatech Cellgro, Manassas, VA)) and cultured in a humidified atmosphere of 5% CO₂ at 37°C.

*In vitro activation of Pmel-1 T cells.* Spleens and lymph nodes were harvested from Pmel-1 TCR transgenic mice (n=2) and cultured with human IL-2 (100 IU/mL, NCI Preclinical Repository, Developmental Therapeutics Program) and hgp10025-33 peptide (1 ug/mL, NH2-KVPRNQDWL-OH, Biosynthesis, Inc., Lewisville, TX) in XVIVO-15 (Lonza, Walkersville, MD) supplemented with 2% FBS. After 72 hours, cells were washed with PBS 1x and cultured in IL-2 and hgp10025-33 peptide at a concentration of 1 × 10⁶ cells/mL.

*In vitro inhibition of PD-1.* Cells were cultured in appropriate media as described above supplemented with 1uM anti-PD-1 Ab (BioXCell, West Lebanon, NH). For the duration of the culture, media was replaced with fresh preparation of media with anti-PD-1 Ab every 24 hours.

*Cytokine*/*chemokine Luminex assay.* Pmel T cells and GL261-gp100 cells were co-cultured at an effector:target (E:T) ratio of 10:1 in complete DMEM media supplemented with 1uM anti-PD-1 Ab. Supernatant was collected at 24 hours later and flash frozen in liquid nitrogen. Analysis with mouse 32-plex cytokine/chemokine Luminex assay performed in collaboration with Dr. Elaine Reed (UCLA).

*xCELLigence real-time cytotoxicity assay.* Cytotoxic killing of tumor cells was assessed with the xCELLigence Real-Time Cell Analyzer System (Acea Biotechnology, San Diego, CA). Target GL261-gp100 cells were plated (10⁵ cells/well) in 150 µL of medium. After overnight tumor cell adherence to the well-bottom, effector cells (Pmel T cells) were added at an E:T ratio of 10:1. For control, maximal cell release obtained with addition of 1% Triton X-100 to additional wells containing only tumor. Cell index (CI) values (relative cell impedance) were collected over 24 hours. Values were normalized to the maximal CI value immediately prior to effector cell plating. The proportion of the normalized CI (nCI) at any time point to the nCI of initial effector cell plating was calculated to delineate percent tumor lysis (23).

*GL261 lysate preparation.* GL261 glioma cells cultured and expanded in complete DMEM media. Cells then harvested and passaged through several freeze-thaw cycles and suspension filtered following. Lysate concentration quantified using Bradford protein assay.

*Bone marrow-derived DC and vaccination.* Preparation of DCs from murine bone marrow progenitor cells were prepared as previously published (Prins et al., Cancer Research 63:8487 (2003)). Briefly, bone marrow cells were cultured in a humidified atmosphere of 5% CO₂ at 37°C overnight in complete RPMI (Mediatech, Inc. Herndon, VA) (supplemented with 10% FBS, 1% (v/v) penicillin and streptomycin). The day following, nonadherent cells were collected and plated with murine interleukin-4 (IL-4, 400 IU/mL, R&D Systems, Minneapolis, MN) and murine granulocyte-macrophage colony stimulating factor (GM-CSF, 100 ng/ml, R&D Systems, Minneapolis, MN). On day 4, adherent cells were re-fed with fresh media with the same cytokines. On day 7, DCs were harvested and resuspended at 1 × 10⁶ cells/ml in complete RPMI and pulsed with GL261 lysate (250 µg/mL). On day 8, DCs were collected and resuspended at 2 × 10⁶ cells/mL in PBS 1x and immediately prepared for injection in 0.2 ml of cell suspension per mouse. Injections were given subcutaneously (s.c.) at 4 sites on the back.

*Intracranial glioma implants.* Female C57BL/6 mice, 6-10 weeks of age, were obtained from our institutional breeding colonies. All mice were bred and kept under defined-flora pathogen-free conditions at the AALAC-approved Animal Facility of the Division of Experimental Radiation Oncology at UCLA. Mice were handled in accordance with the UCLA animal care policy and approved animal protocols. Mice were anaesthetized with an intraperitoneal (i.p.) injection of ketamine/xylazine. After shaving the hair and incising the povidone-swabbed scalp, a burr hole was made in the skull 2.5 mm lateral to bregma using a dental drill with the head of the mouse fixed in a stereotactic apparatus. GL261 glioma cells (2 × 10⁴ in 2 µl PBS) were stereotactically injected with a sterile Hamilton syringe fitted with a 26-gauge needle. The intracranial injection ensued over a 2 min period and at a depth of 3.5 mm below the dura mater. The syringe was retained in the brain for an additional min following complete infusion of cells and then slowly withdrawn to prevent leakage of the cells into leptomeningeal space. Following intracranial tumor implantation, NSG mice were randomized into treatment groups (n=6-16).

*In vivo anti-PD-1 mAb treatment.* Anti-PD-1 mAb administered i.p. at 250 mg/kg (approximately 250 µg/mouse) on appropriate treatment days.

*Tissue harvests, immunohistochemistry, and flow cytometry.* Spleens, lymph nodes, and tumors were harvested from mice on day 21. In cases where sectioning and immunohistochemistry was required, tissue was placed in Zinc Fixative (1x, BD Biosciences, San Jose, CA) for 24 hours and then transferred to 70% ethanol before being embedded in paraffin wax. Spleens and lymph nodes were passed through 70 um cell strainers to generate single-cell suspensions. Lymphocytes were obtained after hypotonic lysis and enumerated using trypan blue exclusion. To determine the number of tumor infiltrating lymphocytes (TILs), tumor-bearing hemispheres were carefully weighed and subsequently minced with a scalpel. The tissue was then placed on a rotator in collagenase with DNase for 24 hours, then lymphocytes isolated using 30%:70% Percoll gradient. Small mononuclear cells within the tumor were enumerated by trypan blue exclusion. Approximately 1×10⁶ lymphocytes were used for staining. TILs were calculated by determining the total number of CD8+ cells per tumor-bearing hemisphere. Fluorochrome conjugated antibodies to CD3, CD4, CD8, CD25, FoxP3, Ly6C, PD-1, and PD-L1 were obtained from Biolegend. All FACS analysis was performed with the use of an LSRII (BD Biosciences). Gates were set based on isotype specific control antibodies (data not shown). Data was analyzed using FlowJo software.

*In vivo PET and MR imaging.* Mice were sedated with 1-3% isoflurane under O₂/N₂ flow and respiration monitored. Mice were kept warm with water heated to 37C circulated using a TP500 water pump (Gaymar Solid State). Tail-vein administration of PET probe was conducted 1 hour prior to scan. PET scanning. Tail-vein catheterization was performed to administer contrast agent (gadolinium, xug/mouse) at appropriate time point during scan. All images were acquired on a 7T Bruker Biospec system with a custom-built 2.2-cm RF birdcage coil. We collected a two-dimensional pre-contrast T1-weighted image using fast low-angle shot (FLASH); a multislice multi-echo (MSME) spin-echo T2-weighted scan for calculation of quantitative T2 maps (16 echoes with TE ranging from 10-160ms in intervals of 10ms), 78um² in-plane resolution and 1mm slice thickness; multiple flip angle 3D FLASH T1-weighted images for calculation of pre-contrast T1 maps using flip angles of 2-20 degrees; and a high-resolution 3D post-contrast T1-weighted image.

## Claims

1. A therapeutic agent in combination with an agent that is a checkpoint inhibitor for use in a method of treating a glioma or initiating, enhancing, or prolonging an anti-glioma response in a subject in need thereof comprising administering to the subject the therapeutic agent in combination with the agent that is a checkpoint inhibitor, wherein the therapeutic agent is a glioma lysate-pulsed dendritic cell vaccine, and wherein the checkpoint inhibitor is selected from the group consisting of a monoclonal antibody, a humanized antibody and a fully human antibody or a combination thereof and inhibits a checkpoint protein selected from the group consisting of PD-1 and PDL1 or a combination thereof

2. The therapeutic agent in combination with an agent that is a checkpoint inhibitor for the use of claim 1, wherein the dendritic cell vaccine is composed of autologous dendritic cells and/or allogenic dendritic cells.

3. The therapeutic agent in combination with an agent that is a checkpoint inhibitor for the use of claim 2, wherein the autologous or allogenic dendritic cells are loaded with glioma lysate prior to administration to the subject.

4. A therapeutic agent in combination with an agent that is a checkpoint inhibitor for use in a method of treating glioma or initiating, enhancing, or prolonging an anti-glioma response in a subject in need thereof comprising administering to the subject the therapeutic agent in combination with the agent that is a checkpoint inhibitor, wherein the therapeutic agent is a dendritic cell vaccine, wherein the dendritic cells of the vaccine are partially matured, and wherein the checkpoint inhibitor is selected from the group consisting of a monoclonal antibody, a humanized antibody and a fully human antibody or a combination thereof and inhibits a checkpoint protein selected from the group consisting of PD-1 and PDL1 or a combination thereof and wherein the partially mature dendritic cells are autologous or allogenic dendritic cells which pick up glioma biomarkers in *situ.*

5. The therapeutic agent in combination with an agent that is a checkpoint inhibitor for the use of any one of the preceding claims, wherein the checkpoint inhibitor and the therapeutic are administered simultaneously or sequentially in either order.

6. The therapeutic agent in combination with an agent that is a checkpoint inhibitor for the use of claim 1, wherein the therapeutic agent is administered prior to the checkpoint inhibitor and wherein the checkpoint inhibitor is a PD-1 inhibitor.

7. The therapeutic agent in combination with an agent that is a checkpoint inhibitor for the use of any one of the preceding claims, wherein the glioma is malignant glioblastoma.

8. The therapeutic agent in combination with an agent that is a checkpoint inhibitor for the use of any one of the preceding claims, further comprising administering a chemotherapeutic agent, targeted therapy or radiation to the subject either prior to, simultaneously with, or after treatment with the combination therapy.

9. The therapeutic agent in combination with an agent that is a checkpoint inhibitor for the use of any one of the preceding claims, wherein the subject has a glioma and the method enhances or prolongs the effects of the checkpoint inhibitor, or enables a subject to respond to the checkpoint inhibitor, or enables the toxicity or the dose of the checkpoint inhibitor to be reduced.

## Patentansprüche

1. Therapeutischer Wirkstoff in Kombination mit einem Wirkstoff, der ein Checkpoint-Inhibitor ist, zur Verwendung in einem Verfahren zum Behandeln eines Glioms oder Initiieren, Verstärken oder Verlängern einer Anti-Gliom-Antwort bei einem Subjekt mit Bedarf daran, umfassend das Verabreichen, an das Subjekt, des therapeutischen Wirkstoffs in Kombination mit dem Wirkstoff, der ein Checkpoint-Inhibitor ist, wobei der therapeutische Wirkstoff ein Gliomlysat-gepulster dendritischer Zellimpfstoff ist und wobei der Checkpoint-Inhibitor ausgewählt ist aus der Gruppe bestehend aus einem monoklonalen Antikörper, einem humanisierten Antikörper und einem vollständig humanen Antikörper oder einer Kombination davon und ein Checkpoint-Protein inhibiert, das ausgewählt ist aus der Gruppe bestehend aus PD-1 und PDL1 oder einer Kombination davon.

2. Therapeutischer Wirkstoff in Kombination mit einem Wirkstoff, der ein Checkpoint-Inhibitor ist, zur Verwendung nach Anspruch 1, wobei der dendritische Zellimpfstoff aus autologen dendritischen Zellen und/oder allogenen dendritischen Zellen zusammengesetzt ist.

3. Therapeutischer Wirkstoff in Kombination mit einem Wirkstoff, der ein Checkpoint-Inhibitor ist, zur Verwendung nach Anspruch 2, wobei die autologen oder allogenen dendritischen Zellen vor der Verabreichung an das Subjekt mit Gliomlysat beladen werden.

4. Therapeutischer Wirkstoff in Kombination mit einem Wirkstoff, der ein Checkpoint-Inhibitor ist, zur Verwendung in einem Verfahren zum Behandeln eines Glioms oder Initiieren, Verstärken oder Verlängern einer Anti-Gliom-Antwort bei einem Subjekt mit Bedarf daran, umfassend die Verabreichen, an das Subjekt, des therapeutischen Wirkstoffs in Kombination mit dem Wirkstoff, der ein Checkpoint-Inhibitor ist, wobei der therapeutische Wirkstoff ein dendritischer Zellilmpfstoff ist, wobei die dendritischen Zellen des Impfstoffs teilweise gereift sind und wobei der Checkpoint-Inhibitor ausgewählt ist aus der Gruppe bestehend aus einem monoklonalen Antikörper, einem humanisierten Antikörper und einem vollständig humanen Antikörper oder einer Kombination davon und ein Checkpoint-Protein inhibiert, das ausgewählt ist aus der Gruppe bestehend aus PD-1 und PDL1 oder einer Kombination davon und wobei die teilweise gereiften dendritischen Zellen autologe oder allogene dendritische Zellen sind, die Gliom-Biomarker *in situ* aufgreifen.

5. Therapeutischer Wirkstoff in Kombination mit einem Wirkstoff, der ein Checkpoint-Inhibitor ist, zur Verwendung nach einem der vorhergehenden Ansprüche, wobei der Checkpoint-Inhibitor und das Therapeutikum gleichzeitig oder sequenziell in beliebiger Reihenfolge verabreicht werden.

6. Therapeutischer Wirkstoff in Kombination mit einem Wirkstoff, der ein Checkpoint-Inhibitor ist, zur Verwendung nach Anspruch 1, wobei der therapeutische Wirkstoff vor dem Checkpoint-Inhibitor verabreicht wird und wobei der Checkpoint-Inhibitor ein PD-1-Inhibitor ist.

7. Therapeutischer Wirkstoff in Kombination mit einem Wirkstoff, der ein Checkpoint-Inhibitor ist, zur Verwendung nach einem der vorhergehenden Ansprüche, wobei das Gliom ein malignes Glioblastom ist.

8. Therapeutischer Wirkstoff in Kombination mit einem Wirkstoff, der ein Checkpoint-Inhibitor ist, zur Verwendung nach einem der vorhergehenden Ansprüche, ferner umfassend das Verabreichen eines chemotherapeutischen Wirkstoffs, einer zielgerichteten Therapie oder einer Bestrahlung an das Subjekt entweder vor, gleichzeitig mit oder nach Behandlung mit der Kombinationstherapie.

9. Therapeutischer Wirkstoff in Kombination mit einem Wirkstoff, der ein Checkpoint-Inhibitor ist, zur Verwendung nach einem der vorhergehenden Ansprüche, wobei das Subjekt ein Gliom hat und das Verfahren die Wirkungen des Checkpoint-Inhibitors verstärkt oder verlängert oder es einem Subjekt ermöglicht, auf den Checkpoint-Inhibitor anzusprechen, oder es ermöglicht, die Toxizität oder die Dosis des Checkpoint-Inhibitors zu reduzieren.

## Revendications

1. Agent thérapeutique combiné à un agent inhibiteur de point de contrôle et destiné à être utilisé dans un procédé de traitement d'un gliome ou d'initiation, d'amélioration ou de prolongation d'une réponse anti-gliome chez un sujet qui en a besoin, le procédé consistant à administrer au sujet l'agent thérapeutique en combinaison avec l'agent inhibiteur de point de contrôle, l'agent thérapeutique étant un vaccin à cellules dendritiques pulsées par un lysat de gliome, et l'inhibiteur de point de contrôle étant choisi dans le groupe constitué d'un anticorps monoclonal, d'un anticorps humanisé et d'un anticorps entièrement humain ou d'une combinaison correspondante et inhibant une protéine de point de contrôle choisie dans le groupe constitué de PD-1 et de PDL1 ou d'une combinaison correspondante.

2. Agent thérapeutique combiné à un agent inhibiteur de point de contrôle et destiné à être utilisé selon la revendication 1, le vaccin à cellules dendritiques étant composé de cellules dendritiques autologues et/ou de cellules dendritiques allogènes.

3. Agent thérapeutique combiné à un agent inhibiteur de point de contrôle et destiné à être utilisé selon la revendication 2, les cellules dendritiques autologues ou allogènes étant chargées avec un lysat de gliome avant l'administration au sujet.

4. Agent thérapeutique combiné à un agent inhibiteur de point de contrôle et destiné à être utilisé dans un procédé de traitement d'un gliome ou d'initiation, d'amélioration ou de prolongation d'une réponse anti-gliome chez un sujet qui en a besoin, le procédé consistant à administrer au sujet l'agent thérapeutique en combinaison avec l'agent inhibiteur de point de contrôle, l'agent thérapeutique étant un vaccin à cellules dendritiques, les cellules dendritiques du vaccin étant partiellement matures, l'inhibiteur de point de contrôle étant choisi dans le groupe constitué d'un anticorps monoclonal, d'un anticorps humanisé et d'un anticorps entièrement humain ou d'une combinaison correspondante et inhibant une protéine de point de contrôle choisie dans le groupe constitué de PD-1 et de PDL1 ou d'une combinaison correspondante, et les cellules dendritiques partiellement matures étant des cellules dendritiques autologues ou allogènes qui captent les biomarqueurs de gliome sur site.

5. Agent thérapeutique combiné à un agent inhibiteur de point de contrôle et destiné à être utilisé selon l'une quelconque des revendications précédentes, l'inhibiteur de point de contrôle et l'agent thérapeutique étant administrés simultanément ou séquentiellement dans n'importe quel ordre.

6. Agent thérapeutique combiné à un agent inhibiteur de point de contrôle et destiné à être utilisé selon la revendication 1, l'agent thérapeutique étant administré avant l'inhibiteur de point de contrôle et l'inhibiteur de point de contrôle étant un inhibiteur de PD-1.

7. Agent thérapeutique combiné à un agent inhibiteur de point de contrôle et destiné à être utilisé selon l'une quelconque des revendications précédentes, le gliome étant un glioblastome malin.

8. Agent thérapeutique combiné à un agent inhibiteur de point de contrôle et destiné à être utilisé selon l'une quelconque des revendications précédentes, consistant en outre à administrer un agent chimiothérapeutique, une thérapie ciblée ou une radiation au sujet avant le traitement, simultanément au traitement ou après le traitement par thérapie combinée.

9. Agent thérapeutique combiné à un agent inhibiteur de point de contrôle et destiné à être utilisé selon l'une quelconque des revendications précédentes, le sujet ayant un gliome et le procédé améliorant ou prolongeant les effets de l'inhibiteur de point de contrôle ou permettant à un sujet de répondre à l'inhibiteur de point de contrôle ou permettant de réduire la toxicité ou la dose de l'inhibiteur de point de contrôle.
